# EUROPÄISCHE PATENTSCHRIFT

(11) **EP 1 653 883 B1**
(45) Veröffentlichungstag und Bekanntmachung des Hinweises auf die Patenterteilung: **21.01.2015**
(21) Anmeldenummer: 04763937.2
(22) Anmeldetag: 10.08.2004
(51) Int. Cl.: A61F 2/07

(54) **STENT ZUR IMPLANTATION IN EIN BLUTGEFÄSS, INSBESONDERE IM BEREICH DES AORTENBOGENS**
STENT FOR IMPLANTATION IN A BLOOD VESSEL, ESPECIALLY IN THE REGION OF THE AORTIC ARCH
STENT DESTINE A L'IMPLANTATION DANS UN VAISSEAU SANGUIN, EN PARTICULIER DANS LA REGION DE LA CROSSE DE L'AORTE

(30) Priorität: 12.08.2003 DE 10337739
(43) Veröffentlichungstag der Anmeldung: 10.05.2006
(73) Patentinhaber: JOTEC GmbH, 72379 Hechingen (DE)
(72) Erfinder: KAUFMANN, Ralf, 72414 Rangendingen (DE); LESMEISTER, Rainer, 72770 Reutlingen (DE); MÜLLER, Hardy, 72406 Bisingen (DE); BRAUN, Michael, 71522 Backnang (DE); GEIS, John, 26160 Bad Zwischenahn (DE)
(74) Vertreter: Laufer, Gabriele
(86) Internationale Anmeldenummer: PCT/EP2004/008916
(87) Internationale Veröffentlichungsnummer: WO 2005/013854

(56) Entgegenhaltungen:
- EP-A- 1 075 825
- WO-A-99/29262
- WO-A-99/39663
- WO-A-99/43378
- WO-A1-97/09945
- DE-A- 10 065 824
- US-A- 6 030 414

## Beschreibung

Die vorliegende Erfindung betrifft einen Stent zur Implantation in ein Blutgefäß, insbesondere im Bereich des Aortenbogens, mit in seiner Längsrichtung hintereinander angeordneten Ringen aus mäanderförmig umlaufenden Stützen und einem an den Ringen befestigten und diese verbindenden Prothesenmaterial, das einen hohlzylindrischen Körper mit umfänglich im Wesentlichen geschlossenen Mantel bildet.

Ein derartiger Stent, allerdings nicht zur Implantation in den Aortenbogen, ist aus dem Stand der Technik bekannt.

Diese auch als endovaskuläre Stents bezeichneten Gefäßstents werden zur Behandlung von Aneurysmen in Arterien implantiert. Unter einem Aneurysma versteht man eine Ausweitung oder Aussackung eines arteriellen Blutgefäßes infolge angeborener oder erworbener Wandveränderungen. Die Aussackung kann dabei die Gefäßwand als Ganzes erfassen oder es kann bei einem sog. falschen Aneurysma Blut aus dem Lumen des Gefäßes zwischen die Gefäßwandschichten treten und diese auseinander scheren. Die Nichtbehandlung eines Aneurysma kann im fortgeschrittenen Stadium zu einer Ruptur der Arterie führen, woraufhin der Patient innerlich verbluten kann.

Aneurysmen treten zwar häufig im Bereich der Baucharterie (Aorta abdominalis) oder Brustarterie (Aorta thoracica) auf, ein Aneurysma kann aber auch im Bereich des aufsteigenden oder absteigenden Astes der Aorta (Aorta ascendens und Aorta descendens) auftreten. Der aufsteigende Ast der Aorta ist unmittelbar mit dem Herzen verbunden. Ausgehend von der Aortenwurzel (Sinus aortae) verläuft der aufsteigende Ast in leicht gekrümmter Form vom Herzen weg nach oben und geht dort in den Aortenbogen (Arcus aortae) über. Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit dem absteigenden Ast.

Die WO 99/43378 A offenbart ein Einführungsystem zur Freisetzung einer Stent-Baugruppe sowie eine endovaskuläre Baugruppe eines mehrteiligen Stentgrafts, wobei dieser Stentgraft einen Verankerungsstent sowie einen Stützstent aufweist, die von einem Prothesenmaterial, d.h. einem Mantel umgeben sind. Der Stützstent, dessen Ringe untereinander verbunden sind, wird dabei zwischen den beiden Ringen des Verankerungsstents eingeführt, so dass die Ringe des Verankerungsstent die äußeren Ringe an den jeweiligen Enden des Stents bilden. Der Verankerungsstent und der Stützstent werden durch das Prothesenmaterial zusammengehalten.

Die EP-A-1 075 825 offenbart eine verzweigte intraaortale Prothese, die in die Bauchaorta eines menschlichen Körpers implantiert wird, um ein Aneurysma an der Aortengabelung zu behandeln. Die Prothese weist eine Struktur mit einem Hauptstamm auf, der sich in zwei Schenkel von gleicher Länge teilt, sowie eine Umhüllung, die die Schenkel vollständig und den Hauptstamm teilweise umgibt. Die Prothese kann ferner röntgendichte Marker aufweisen, die in einem V-förmigen Muster in der netzförmigen Struktur des Stents angebracht sind.

Die WO 97/09945 offenbart einen expandierbaren endovaskulären Stent mit einem flexiblen tubulären Körper mit einer länglichen Achse, dessen Wand durch miteinander verbundene, geschlossene Rahmenzellen gebildet wird, von denen jeweils mindestens zwei miteinander im umlaufende Richtung verbunden sind. Die Zellen bilden dabei eine Herz- oder Pfeilform, wobei der Stent insgesamt auch von einem Prothesenmaterial umgeben sein kann.

Aus der DE 100 65 824 A1, die den nächstliegende Stand der Technik gegenüber dem Gegenstand des Anspruchs 1 bildet, ist ein Stent zur Implantation in den aufsteigenden Ast der Aorta bekannt. Der bekannte Stent weist einen hohlen, in Längsrichtung für den Durchgang von Blut offenen, hohlzylindrischen Körper mit einer Wand auf, die durch eine Maschenstruktur gebildet ist. Der Körper des bekannten Stents ist der anatomischen Form der Aortenwurzel angepasst und sich konkav erweiternd ausgebildet. An seinem dem Herzen zugewandten proximalen Ende sind an dem Stent in Umfangsrichtung verteilt schmale Fixierelemente angeordnet. An seinem in den Aortenbogen hineinreichenden distalen Ende ist der Körper des bekannten Stents sozusagen schräg abgeschnitten, so dass der Stent auf seinem Umfangsbereich, der im implantierten Zustand von den Kopfgefäßen weggelegen ist, eine größere Längenausdehnung aufweist als an dem gegenüberliegenden Umfangbereich. Auf diese Weise wird vermieden, dass die vom Aortenbogen abzweigenden Kopfgefäße durch den Stent abgedeckt werden.

Der bekannte Stent hat sich als im Bereich der Aorta ascendens gut einsetzbar erwiesen, auf Grund der völlig anders gelagerten anatomischen Verhältnisse im Bereich der Aorta descendens kann jedoch ein derart ausgebildeter Stent nicht verwendet werden, um Aneurysmen im Bereich der Aorta descendens zu behandeln.

Bei derartigen thorakalen Aortenaneurysmen, die sich ggf. bis in den Aortenbogen, also bis zur linken Schlüsselbeinarterie (A. subclavia sinistra) ausdehnen, besteht nämlich das Problem, dass für die Stents nicht genügend proximale Fixierungs- und Dichtungsfläche vorhanden ist. Mit anderen Worten, der bekannte Stent kann im Aortenbogen gegenüber und proximal des Abganges der A. subclavia sinistra nicht hinreichend fixiert werden.

In einem solchen Fall sind deshalb vor der endoluminalen Implantation einer Gefäßendoprothese, also eines entsprechenden Stents, chirurgische Eingriffe erforderlich. Dabei wird vor der Implantation des Stents eine gefäßchirurgische Verbindung zwischen der proximal von der A. subclavia sinistra vom Aortenbogen abgehenden A. carotis communis und der A. subclavia sinistra geschaffen, so dass die Schlüsselbeinarterie sozusagen über die A. carotis communis versorgt wird. Der Abgang der Schlüsselbeinarterie vom Aortenbogen kann dann problemlos von einer Gefäßendoprothese überdeckt und durch diese verschlossen werden, um für eine ausreichende Fixierungs- und Dichtungsfläche an der Aorteninnenwand zu sorgen.

Derartige gefäßchirurgische Eingriffe sind sehr zeitaufwendig, zudem muss der Patient an eine Herz-Lungen-Maschine angeschlossen und die Körpertemperatur des Patienten stark heruntergekühlt werden. Aus diesem Grund liegt die Mortalitätsrate bei derartigen Eingriffen sehr hoch.

Ein weiterer Nachteil ist darin zu sehen, dass eine Notversorgung eines Patienten mit thorakalem Aortenaneurysma durch Einführen eines Stents auf minimal-invasivem Wege bisher quasi nicht möglich ist, da eine hinreichend sichere Fixierung des proximalen Endes des Stents Probleme bereitet.

Vor diesem Hintergrund liegt der vorliegenden Erfindung die Aufgabe zugrunde, einen Stent der eingangs genannten Art zu schaffen, der mit seinem proximalen Ende im Bereich des Aortenbogens platziert werden kann.

Bei dem eingangs genannten Stent wird diese Aufgabe erfindungsgemäß dadurch gelöst, dass nur zwischen dem letzten und dem vorletzten Ring am proximalen Ende des Stents zumindest eine V-förmige Verbindungsstütze mit zwei Schenkeln, die sich V-förmig zum distalen Ende des Stents erweitern, vorgesehen ist, wobei die V-förmige Verbindungsstütze die beiden Ringe miteinander verbindet, und dass die Ringe im proximalen Bereich lediglich über Prothesenmaterial miteinander verbunden sind.

Die der Erfindung zugrundeliegende Aufgabe wird auf diese Weise vollkommen gelöst.

Die Erfinder der vorliegenden Anmeldung haben nämlich erkannt, dass auch Stents mit einem Mantel aus Prothesenmaterial zur Implantation in die A. descencens geeignet sind, wenn nur zwischen dem letzten und dem vorletzten Ring eine Verbindungsstütze vorgesehen, die diese beiden Ringe miteinander verbindet. Bei dem zur Implantation in die A. ascendens geeigneten Stent gemäß DE 100 65 824 ist das im Aortenbogen liegende distale Ende des Stents abgeschrägt und ggf. offenmaschig, um eine Versorgung der Kopfarterien zu gewährleisten. Ein derartiger Maschenstent ist zur Behandlung von Aneurysmen im Bereich der Aorta descendens nicht geeignet, wobei auch eine reine Abschrägung am im Aortenbogen liegenden proximalen Ende des Stents zwar die Versorgung der abgehenden Arterien ermöglicht, nicht jedoch eine hinreichende Fixierung. Die Verbindungsstütze ermöglicht es jetzt dagegen, zwischen dem letzten und dem vorletzten Ring bspw. einen Teil der Mantelfläche auszusparen oder den Mantel mit Löchern zu versehen, so dass die Versorgung der abgehenden Kopfarterien gesichert ist. Andererseits sorgen sowohl der letzte Ring als auch die Verbindungsstützen dafür, dass das proximale Ende des neuen Stents sicher im Aortenbogen fixiert wird.

Dabei ist es bevorzugt, wenn zwischen dem letzten und dem vorletzten Ring im Bereich der Verbindungsstütze ein im Wesentlichen von Prothesenmaterial freier Mantelbereich aufgespannt ist, und wobei vorzugsweise der im Wesentlichen vom Prothesenmaterial freie Mantelbereich sich zum proximalen Ende des Stents keilförmig aufweitet.

Hier ist von Vorteil, dass zum einen nicht der gesamte Mantelbereich zwischen dem letzten und dem vorletzten Ring ohne Prothesenmaterial ausgebildet ist. Dadurch ist es möglich, an der von den Abgängen der Kopfarterien abgelegenen Innenwand des Aortenbogens Prothesenmaterial und damit einen Mantelbereich vorzusehen, der nicht nur zur Abstützung, sondern auch als Dichtungsfläche verwendet wird. Durch die V-förmige Ausbildung der Verbindungsstütze lässt sich der sich zum proximalen Ende des Stents keilförmig aufweitende, von Prothesenmaterial freie Mantelbereich besonders einfach realisieren. Der Mantel ist im Bereich des keilförmigen Mantelbereiches entfernt, wobei die sich so ergebenden Kanten des Prothesenmaterials an den V-förmigen Streben der Verbindungsstütze befestigt werden.

Während im distalen Bereich des neuen Stents die einzelnen Ringe miteinander lediglich über den Mantel, also das Prothesenmaterial, verbunden sind, erfolgt die Verbindung zwischen dem letzten und dem vorletzten Ring zum einen ebenfalls über den Mantel, andererseits aber auch über die Verbindungsstütze. Die Verbindungsstütze sorgt dafür, dass trotz des teilweise entfernten Mantels der letzte Ring am proximalen Ende sicher mit dem restlichen hohlzylindrischen Körper des Stents verbunden ist.

Allgemein ist es bevorzugt, wenn die Stützen einen Z-förmigen Verlauf mit abwechselnd zum proximalen und zum distalen Ende des Stents weisenden Spitzbögen aufweisen, die durch schräg zur Längsrichtung verlaufende Stützabschnitte miteinander verbunden sind.

Auf diese Weise wird eine Stentstruktur geschaffen, die einen hinreichenden radialen Druck ausübt, um sich in den proximal und distal vom Aneurysma liegenden Gefäßbereichen zu verankern, wobei andererseits der Stent dem Druck durch das durch ihn hindurchfließende Blut standhalten kann.

Dabei ist es bevorzugt, wenn die proximalen Spitzbögen des letzten Ringes zu den proximalen Spitzbögen des vorletzten Ringes einen Abstand aufweisen, der größer ist als der Abstand zwischen den proximalen Spitzbögen des vorletzten Ringes und den proximalen Spitzbögen des vorvorletzten Ringes.

Mit anderen Worten, der letzte Ring weist einen größeren Abstand zum vorletzten Ring auf als die anderen Ringe im Stent untereinander.

Dies hat den Vorteil, dass sich der Stent an seinem proximalen Ende leichter der Krümmung im Aortenbogen anpassen kann. Dies wird zwar zum einen bereits durch den im Wesentlichen von Prothesenmaterial freien Mantelbereich ermöglicht, durch den größeren Abstand zwischen den Ringen am proximalen Ende wird hier die Steifigkeit jedoch stärker durch das verbleibende Prothesenmaterial bestimmt als im sonstigen Bereich des Stents, was eine bessere Biegsamkeit ermöglicht.

Weiter ist es bevorzugt, wenn die proximalen Spitzbögen des letzten Ringes zu den distalen Spitzbögen des vorletzten Ringes einen Abstand aufweisen, der größer ist als der Abstand zwischen dem Abgang der A. subclavia sinistra und dem Abgang der A. carotis communis vom Aortenbogen.

Bei dieser Maßnahme ist von Vorteil, dass die proximalen Spitzbögen des letzten Ringes sich proximal von der A. carotis communis an die Innenwand des Aortenbogens anlegen können, während die distalen Spitzbögen des vorletzten Ringes sich distal von der A. subclavia sinistra innen an die Aortenwand anlegen. Damit wird zum einen für eine sichere Verankerung des proximalen Endes des neuen Stents im Aortenbogen gesorgt, wobei gleichzeitig verhindert wird, dass im Bereich der Abgänge von A. carotis communis und A. subclavia sinistra ein zu starker Druck auf die Aorteninnenwand ausgeübt wird. Ferner ermöglicht es diese Konstruktion, den im Wesentlichen von Prothesenmaterial freien Mantelbereich so zu wählen, dass die Abgänge von A. carotis communis und A. subclavia sinistra nicht von Prothesenmaterial bedeckt werden.

Allgemein ist es bevorzugt, wenn ein zum distalen Ende des Stents weisender Spitzbogen des vorletzten Ringes mit einem Spitzbogen einer Verbindungsstütze in Anlage ist, wobei vorzugsweise der zum distalen Ende des Stents weisende Spitzbogen des vorletzten Ringes zumindest teilweise durch den Spitzbogen der Verbindungsstütze gebildet ist.

Hier ist von Vorteil, dass der Z-förmige Verlauf des vorletzten Ringes durch die Verbindungsstütze nicht verändert wird, die Verbindungsstütze passt sich vielmehr in die übliche Struktur des Ringes ein. Dabei kann der distale Spitzbogen des vorletzten Ringes teilweise weggelassen werden, wobei die so entstehende Lücke durch den Spitzbogen der Verbindungsstütze ausgefüllt ist.

Weiter ist es bevorzugt, wenn die Verbindungsstütze zwei Schenkel aufweist, die an ihrem proximalen Ende jeweils mit einem Stützabschnitt des letzten Ringes in Anlage sind.

Hier ist von Vorteil, dass auch der Z-förmige Verlauf des letzten Ringes durch die Verbindung mit der Verbindungsstütze nicht verändert werden muss.

Dabei ist es weiter bevorzugt, wenn die Verbindungsstütze zwei Schenkel aufweist, die an ihrem distalen Ende jeweils mit einem Stützabschnitt des vorletzten Ringes in Anlage sind.

Wie bereits erwähnt, hat diese Maßnahme konstruktive Vorteile, der Z-förmige Verlauf des letzten und vorletzten Ringes wird nicht gestört.

Ein weiterer Vorteil liegt darin, dass durch diese Struktur ein Druck auf den letzten Ring dazu führt, dass sich die V-förmige Verbindungsstütze nach außen aufspreizt, so dass das dort befestigte Prothesenmaterial nach außen an die Aorteninnenwand gedrückt wird und so eine Abdichtung des Blutstromes gegen das Aneurysmavolumen bildet. Der nicht von Prothesenmaterial bedeckte Bereich der Stütze des proximal letzten Ringes sorgt dabei also für eine Verbiegung des von Prothesenmaterial bedeckten proximalen Prothesenendes entlang der torusförmigen Aorteninnenwand und gleichzeitig für ein Aufspreizen des keilförmigen ungedeckten Bereiches gegenüber den supraaortalen Gefäßabgängen sowie für das Aufdrücken der Schenkel der Verbindungsstütze zur Abdichtung des Blutstromes gegen das Aneurysmavolumen.

Bei den oben stehenden Ausführungsformen des erfindungsgemäßen Stents kann dabei die Anzahl der Spitzbögen des letzten Rings gleich derjenigen des vorletzten Rings sein.

In einer anderen Ausführungsform ist es bevorzugt, wenn die Anzahl der Spitzbögen des letzten Ringes geringer ist als die Anzahl der Spitzbögen des vorletzten Ringes.

Der Stent, bzw. die Verteilung der Spitzbögen im letzten Ring des Stents, kann dabei bspw. derart ausgebildet sein, dass in dem im Wesentlichen von Prothesenmaterial freien Mantelbereich, der im Bereich der Verbindungsstütze aufgespannt ist, kein freier distaler Spitzbogen vorliegt. Dadurch kann sich der Stent bei dieser Ausführungsform beim Zurückziehen in die distale Richtung nicht in den Gefäßwänden verhaken.

Diese Ausführungsform kann ferner bspw. derart ausgebildet sein, dass im letzten Ring an derjenigen Stelle kein distaler Spitzbogen ausgebildet ist, die dem zum distalen Ende des Stents weisenden Spitzbogen des vorletzten Rings, der zumindest teilweise durch den Spitzbogen der Verbindungsstütze gebildet ist, entspricht bzw. gegenüberliegt.

In wiederum einer anderen Ausführungsform ist bevorzugt, wenn zumindest zwei weitere Verbindungsstützen vorgesehen sind, die jeweils mit ihrem proximalen Ende mit einem Stützabschnitt des letzten Ringes und jeweils mit ihrem distalen Ende mit einem Stützabschnitt des vorletzten Ringes in Anlage sind.

Dabei ist in einer weiteren Ausführungsform ferner bevorzugt, wenn sich die zwei weiteren Verbindungsstützen in einem Punkt kreuzen.

Durch die weiteren Verbindungsstützen dieser Ausführungsformen kann die Flankenstabilität und die Flankendichtigkeit vorteilhaft erhöht werden.

Diese Ausführungsformen haben ferner zum Vorteil, dass die Stabilität des Stents insbesondere im distalen Bereich unterstützt wird, wodurch ebenfalls Verhakungsproblematiken der Spitzbögen beim Zurückziehen des Stents in distale Richtung vermieden werden können.

Weiter ist es bevorzugt, wenn der letzte Ring und die Verbindungsstütze einstückig miteinander ausgebildet sind.

Diese Maßnahme ist konstruktiv von Vorteil, es ist lediglich erforderlich, einen üblichen Z-förmigen Ring zu biegen und dann eines der freien Enden in Richtung des distalen Bereiches zu verlängern, dort umzulenken und zu dem letzten Ring zurückzuführen.

Allgemein ist es bevorzugt, wenn der jeweilige Stützabschnitt des letzten oder vorletzten Ringes und ein mit diesem in Anlage befindlicher Abschnitt der Verbindungsstütze durch eine Presshülse miteinander verbunden sind.

Diese Maßnahme ist zum einen konstruktiv von Vorteil, denn die aneinander anliegenden Drahtbereiche werden auf diese Weise miteinander verkrimpt. Diese Maßnahme ist aber auch unter Sicherheitsaspekten von Vorteil, denn auf diese Weise werden die freien Enden sowohl des vorletzten als auch des letzten Ringes durch die Krimphülsen abgedeckt, so dass Verletzungen der Aorteninnenwand vermieden werden.

Weiter ist es bevorzugt, wenn die zum proximalen Ende des Stents weisenden Spitzbögen des letzten Ringes gegenüber dessen zum distalen Ende des Stents weisenden Spitzbögen nach außen gebogen sind.

Bei dieser Maßnahme ist von Vorteil, dass sich die proximalen Spitzbögen der Innenwand des Aortenbogens anpassen, die in guter Näherung als Innenfläche eines Torus-Segmentes angesehen werden kann. Gleichzeitig erzeugen die nach außen gebogenen Spitzbögen durch die Elastizität des Drahtmaterials einen Druck auf die Aortenwand, der die Fixierung und Positionierung sowie die Dichtwirkung der Gefäßendoprothese, also des Stents, - sicherstellt.

Allgemein ist es bevorzugt, wenn die Stützen und die Verbindungsstütze aus einem drahtförmigen, elastischen Material bestehen.

Diese Maßnahme ist an sich bekannt, sie sorgt dafür, dass der Stent zur Einführung in das Lumen der Aorta zunächst zusammengedrückt werden kann, so dass sich sein Außendurchmesser verringert. Nach dem Freisetzen des Stents expandiert sich dieser und verankert sich so in dem entsprechenden Blutgefäß.

Allgemein ist es bevorzugt, wenn distal zu einem distalen Spitzbogen der Verbindungsstütze an dem Mantel ein distaler Marker angebracht ist, wobei ferner vorzugsweise distal zu einem Stützabschnitt des letzen Ringes an dem Mantel ein proximaler Marker angeordnet ist, wobei die Marker vorzugsweise Röntgenmarker sind.

Bei dieser Maßnahme ist von Vorteil, dass eine Positionierungshilfe geschaffen wird, durch die während der Implantation und zur Überprüfung der Lage des im Wesentlichen von Prothesenmaterial freien Mantelbereiches gegenüber den supraaortalen Abgängen nach der Implantation die aktuelle Lage des Stents verfolgt werden kann.

Allgemein ist es bevorzugt, wenn das Prothesenmaterial aus textilem Material oder aus Folie besteht, wobei weiter vorzugsweise das Prothesenmaterial an den Stützen und der Verbindungsstütze durch Nähen, Kleben oder Einschmelzen befestigt ist.

Diese Maßnahmen sind aus dem Stand der Technik bekannt, sie ermöglichen eine schnelle und preiswerte, aber dennoch sichere Fertigung des neuen Stents.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in Bezug auf diese nachstehend näher beschrieben. Es zeigen:
- Fig. 1: eine schematische Darstellung einer Ausführungsform eines in den absteigenden Ast der Aorta implantierten endovaskulären Stents;
- Fig. 2: die Stützstruktur für den Stent aus Fig. 1, jedoch ohne den umgebenden Mantel;
- Fig. 3: den proximal letzten und vorletzten Ring des Stents aus Fig. 2, noch ohne Verbindung untereinander;
- Fig. 4: die beiden Ringe aus Fig. 3, jetzt jedoch durch Presshülsen miteinander verbunden;
- Fig. 5: eine weitere Ausführungsform des erfindungsgemäßen Stents ohne umgebenden Mantel;
- Fig. 6: noch eine weitere Ausführungsform des erfindungsgemäßen Stents mit zusätzlichen Verbindungsstützen, ebenfalls ohne umgebenden Mantel;
- Fig. 7a: den proximal letzten und vorletzten Ring des Stents aus Fig. 6, mit den anzubringenden Verbindungsstützen, jedoch noch ohne die Verbindung untereinander;
- Fig. 7b: die beiden Ringe aus Fig. 7a, jetzt durch Presshülsen untereinander verbunden;
- Fig. 8: wiederum eine weitere Ausführungsform des erfindungsgemäßen Stents mit sich kreuzenden zusätzlichen Verbindungsstützen, ohne umgebenden Mantel;
- Fig. 9a: den proximal letzten und vorletzten Ring des Stents aus Fig. 8, mit den anzubringenden, sich kreuzenden Verbindungsstützen, noch ohne Verbindung untereinander; und
- Fig. 9b: die beiden Ringe aus Fig. 9a, jetzt durch Presshülsen miteinander verbunden.

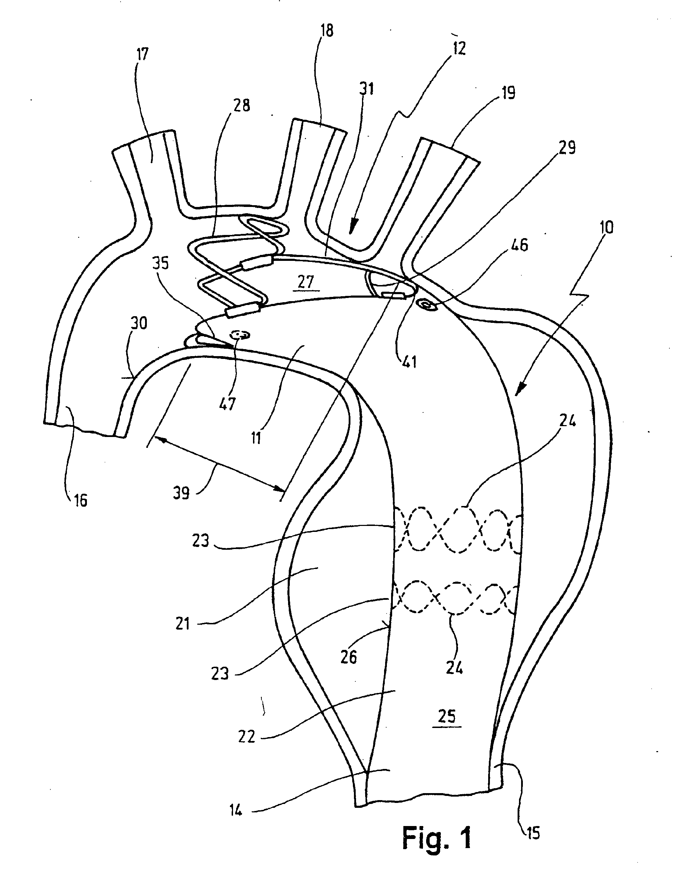

In Fig. 1 ist mit 10 ein Stent gezeigt, der mit seinem proximalen Ende 11 in dem Aortenbogen 12 und mit seinem distalen Ende 14 in der Aorta descendens 15 verankert ist.

Bevor der Stent 10 näher beschrieben wird, soll zunächst das in Fig. 1 ebenfalls schematisch dargestellte Aortensystem erläutert werden.

Der aufsteigende Ast 16 (A. ascendens) der Aorta ist über die in Fig. 1 nicht gezeigte Aortenwurzel (Sinus aortae) mit der ebenfalls nicht dargestellten linken Kammer des Herzens verbunden. Die Aorta ascendens 16 ist über den Aortenbogen 12 mit der Aorta descendens 15 verbunden. Im Bereich des Aortenbogens 12 gehen arterielle Kopfgefäße ab, nämlich der Arterienstamm Truncus brachiocephalicus 17, die Arteria carotis communis 18 und die Arteria subclavia sinistra 19.

In der Aorta descendens 15 ist bei 21 ein Aneurysma dargestellt, das durch den Stent 10 sozusagen überbrückt ist. Der aus der Aorta ascendens 16 kommende Blutstrom gelangt über den Aortenbogen 12 in das proximale Ende 11 des Stents 10 und verlässt diesen am distalen Ende 14. Zu diesem Zweck weist der Stent 10 einen hohlzylindrischen Körper 22 auf, der durch in Fig. 1 schematisch angedeutete Ringe 23 aus mäanderförmigen Stützen 24 gebildet ist, die durch Prothesenmaterial 25 miteinander verbunden sind. Das Prothesenmaterial 25 in bekannter Weise ein textiles Material oder eine Folie und ist durch Nähen, Kleben oder Einschmelzen an den Stützen 24 befestigt.

Auf diese Weise wird der Durchgang durch den Stent offen gehalten, so dass sich der hohlzylindrische Körper 22 bildet.

An seinem proximalen Ende 11 weist der Stent 10 einen keilförmigen, sich zum proximalen Ende 11 aufweitenden, freien Mantelbereich 27 auf, der zwischen einem proximal letzten Ring 28, einem proximal vorletzten Ring 29 sowie einer Verbindungsstütze 31 aufgespannt ist, die den letzten Ring 28 mit dem vorletzten Ring 29 verbindet.

Dieser im Wesentlichen von Prothesenmaterial 25 freie Mantelbereich 27 ermöglicht es, dass aus der Aorta ascendens 16 herangeführtes Blut in die Arteria carotis communis 18 sowie die Arteria subclavia sinistra 19 gelangen kann. Außerhalb des freien Mantelbereiches 27 befindet sich jedoch zwischen dem letzten Ring 28 und dem vorletzten Ring 29 Prothesenmaterial 25, so dass sich der Stent 10 mit seinem proximalen Ende hier dicht an die bei 30 angedeutete Aorteninnenwand anschmiegt. Auf diese Weise wird verhindert, dass Blut zwischen den Stent 10 und der Aorteninnenwand 30 hindurch in den Bereich des Aneurysmas 21 gelang, dieses ggf. weiter aufdehnt und schließlich zu einer Ruptur führt.

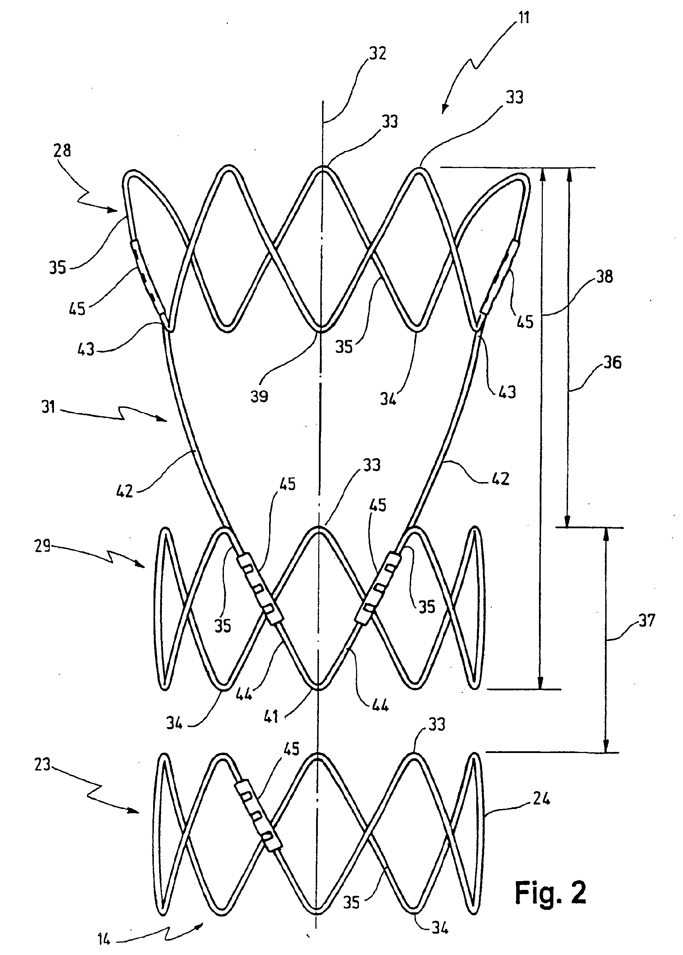

Der konstruktive Aufbau des Stents 10 aus Fig. 1 ist in der schematischen Seitenansicht der Fig. 2 näher dargestellt, wobei aus Gründen der Übersichtlichkeit das Prothesenmaterial 25 in Fig. 2 weggelassen wurde.

Zunächst ist zu erkennen, dass in Längsrichtung 32 hintereinander der letzte Ring 28, der vorletzte Ring 29 sowie weitere Ringe 23 angeordnet sind, von denen lediglich einer dargestellt ist. Jeder dieser Ringe 23, 28, 29 weist mehrere proximale Spitzbögen 33 sowie distale Spitzbögen 34 auf, die durch schräg zur Längsrichtung 32 verlaufende Stützabschnitte 35 miteinander verbunden sind. Auf diese Weise bilden sich die aus mäanderförmig umlaufenden Stützen 24 bestehenden Ringe 23, 28 und 29. Es sei noch erwähnt, dass sowohl die Stützen 24 als auch die Verbindungsstütze 31 aus einem drahtförmigen, elastischen Material bestehen.

Die proximalen Spitzbögen 33 des letzten Ringes 28 weisen zu den proximalen Spitzbögen 33 des vorletzten Ringes 29 einen bei 36 angedeuteten Abstand auf, der größer ist als der Abstand 37 zwischen den proximalen Spitzbögen 33 des vorletzten Ringes 29 und den proximalen Spitzbögen 33 des vorvorletzten Ringes 23. Auf diese Weise ist der Stent 10 an seinem proximalen Ende 11 biegsamer und beweglicher als in Richtung seines distalen Endes 14.

Ferner weisen die proximalen Spitzbögen 33 des letzten Ringes 28 zu den distalen Spitzbögen 34 des vorletzten Ringes 29 einen Abstand 38 auf, der größer ist als der in Fig. 1 angedeutete Abstand 39 zwischen dem Abgang der Arteria subclavia sinistra 19 und dem Abgang der Arteria carotis communis 18 vom Aortenbogen 12. Auf diese Weise kann sich der proximal letzte Ring 28 proximal von der Arteria carotis communis an der Aorteninnenwand 30 verspannen, während sich der vorletzte Ring 29 distal zu der Arteria subclavia sinistra verspannen und dort das Prothesenmaterial 25 auf dem gesamten Umfang gegen die Aorteninnenwand 30 drücken kann.

Die V-förmige Verbindungsstütze 31, die den letzten Ring 28 und den vorletzten Ring 29 miteinander verbindet, weist einen distalen Spitzbogen 41 sowie zwei sich an den Spitzbogen 41 anschließende Schenkel 42 auf, die sich V-förmig zum distalen Ende 11 des Stents 10 erweitern. Die Schenkel 42 sind mit ihren proximalen Enden 43 in Anlage mit Stützabschnitten 35 des letzten Ringes 28. Mit ihren distalen Enden 44 sind die Schenkel 42 in Anlage mit entsprechenden Stützabschnitten 35 des vorletzten Ringes 29. Die Verbindung zwischen den Stützabschnitten 35 und den jeweiligen Enden 43, 44 der Schenkel 42 erfolgt über Presshülsen 45, die auch die mäanderförmigen Stützen 24 bspw. des Ringes 23 zu ihrem Z-förmigen Verlauf schließen.

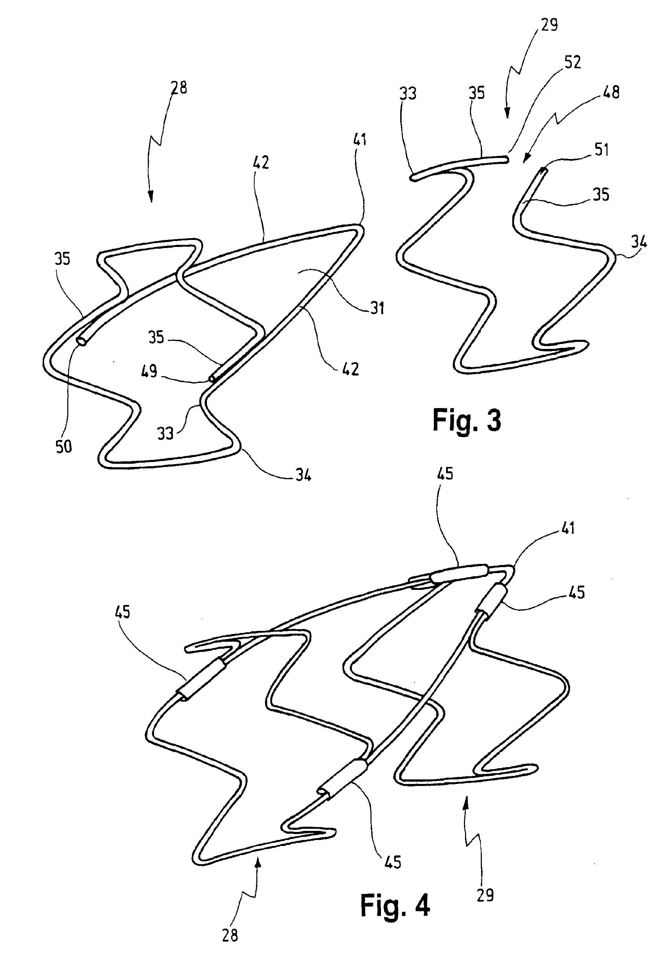

In Fig. 3 sind der letzte Ring 28 sowie der vorletzte Ring 29 unmittelbar nach der Fertigung, also noch vor der Verbindung miteinander dargestellt.

Es ist in Fig. 3 links zu erkennen, dass der letzte Ring 28 und die Verbindungsstütze 31 einstückig miteinander ausgebildet sind. Ferner ist in Fig. 3 rechts zu erkennen, dass dem vorletzten Ring 29 sozusagen ein distaler Spitzbogen 34 fehlt, was durch eine bei 48 angedeutete Lücke dargestellt ist. Diese Lücke wird jetzt durch den distalen Spitzbogen 41 der Verbindungsstütze 31 gefüllt.

Der letzte Ring 28 wird beginnend bei seinem freien Ende 49 durch mehrfaches Z-förmiges Umbiegen des entsprechenden Drahtmaterials gebildet, so dass sich die proximalen und distalen Spitzbögen 33 und 34 bilden. Wenn der Ring 28 geschlossen ist, wird das Drahtmaterial distal weitergeführt und am distalen Spitzbogen 41 umgebogen und zurückgeführt zum Stützabschnitt 35, wo der Draht abgeschnitten wird, so dass sich das zweite freie Ende 50 des einstückigen Gebildes ergibt, das sowohl den letzten Ring 28 als auch die Verbindungsstütze 31 darstellt. Der vorletzte Ring 29 weist entsprechend zwei freie Enden 51 und 52 auf, die beiderseits der Lücke 48 liegen.

Der letzte Ring 28 sowie der vorletzte Ring 29 werden jetzt so zusammengeführt, wie dies in Fig. 4 dargestellt ist, wobei an den freien Enden 49 bis 52 jeweils eine Presshülse 45 aufgeschoben wird, die zum einen die Schenkel 42 mit den entsprechenden Stützabschnitten 35 verbindet und zum anderen die freien Enden 49 bis 52 verdeckt, so dass eine Verletzung der Aorteninnenwand vermieden wird.

Der letzte Ring 28 sowie der vorletzte Ring 29 bildet zusammen mit der Verbindungsstütze 31 eine Fixierungsstruktur, die am proximalen Ende 11 des Stents 10 vorgesehen ist. Mit dieser Fixierungsstruktur kann nun auch die Aorteninnenwand gegenüber und proximal des Abganges der Arteria subclavia sinistra als Fixierungs- und Dichtungsfläche genutzt werden, ohne dass dieser Abgang verschlossen wird. Wenn der neue Stent in dem dafür vorgesehenen Bereich implantiert wird, ist es jetzt nicht mehr erforderlich, vorher einen belastenden Serviceeingriff durchzuführen.

Es sei noch erwähnt, dass die proximalen Spitzbögen 33 des letzten Ringes 28 gegenüber den distalen Spitzbögen 34 nach außen aufgeweitet bzw. nach außen gebogen sind, was in Fig. 2 oben zu erkennen ist. Dadurch passen sich die Spitzbögen 33 der Aorteninnenwand 30 an und erzeugen gleichzeitig durch die Elastizität des Drahtmaterials einen Druck auf die Aorteninnenwand 30, der die Fixierung und Positionierung sowie die Dichtwirkung der Gefäßendoprothese sicherstellt.

In Fig. 1 ist ferner zu erkennen, dass der letzte Ring 28 nach vorne gebogen werden kann, wodurch sich die Schenkel 42 der Verbindungsstütze 31 nach außen spreizen und eng an die Aorteninnenwand 30 anlegen. Dies wird noch unterstützt durch den Druck, den die Aorteninnenwand auf den letzten Ring 28 ausübt, der unter Druck ebenfalls die Schenkel 42 der Verbindungsstütze 31 nach außen spreizt. Auf diese Weise wird das dort befestigte Prothesenmaterial nach außen an die Aorteninnenwand 30 gedrückt und bildet so eine Abdichtung des Blutstromes gegen das Volumen des Aneurysma 21.

Der keilförmig vom Prothesenmaterial 25 frei bleibende Bereich 27 sorgt für die entsprechende Versorgung von Arteria subclavia sinistra 19 und Arteria carotis communis 18.

Als Positionierungshilfe während der Implantation und zur Überprüfung der Lage des ungedeckten Prothesenbereiches, also des freien Mantelbereiches 27 gegenüber den supraaortalen Abgängen 19 und 18 nach der Implantation dienen zwei Röntgenmarker 46 und 47, die in Fig. 1 zu sehen sind. Der Marker 46 ist ein distaler Marker, der distral zu dem distalen Spitzbogen 41 der Verbindungsstütze 31 angeordnet ist. Der Marker 47 ist dagegen ein proximaler Marker, der distal zu einem Stützabschnitt des letzten Ringes 28 an dem Mantel 26 befestigt ist.

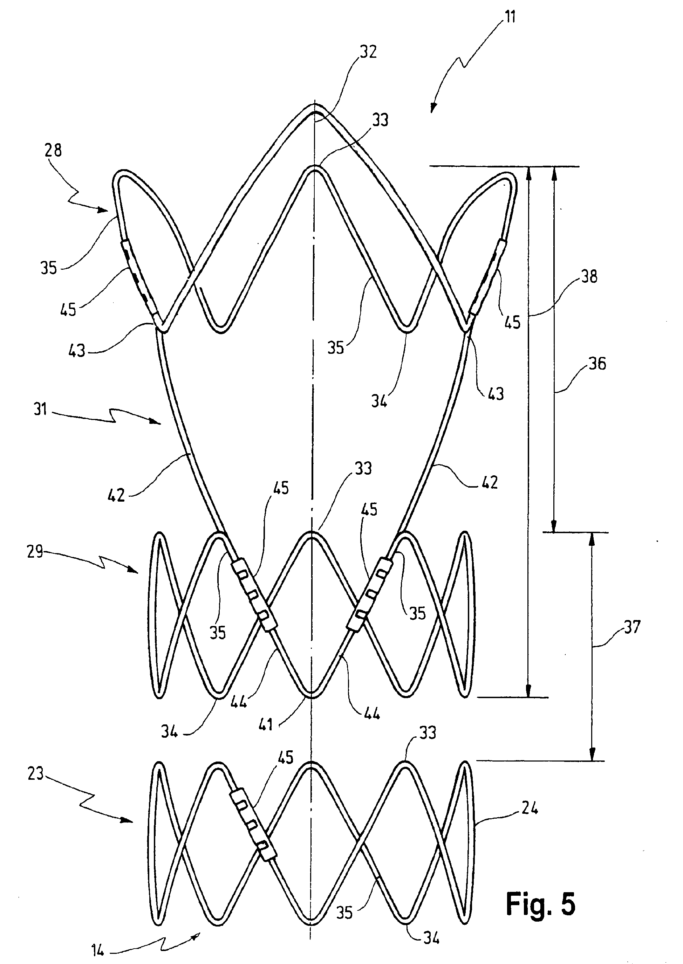

Eine weitere Ausführungsform des erfindungsgemäßen Stents ist in einer schematischen Seitenansicht in Fig. 5 dargestellt, wobei auch hier - wie in Fig. 2 - aus Gründen der Übersichtlichkeit das Prothesenmaterial 25 weggelassen wurde. In den Fig. 5 bis 9a wurden zur Bezeichnung der gleichen Elemente der Stents die gleichen Bezugszeichen wie in Fig. 2 verwendet.

In Fig. 5 ist zu erkennen, dass auch bei dieser Ausführungsform in Längsrichtung 32 hintereinander der letzte Ring 28, der vorletzte Ring 29 sowie weitere Ringe 23 angeordnet sind, von denen lediglich einer dargestellt ist. Es ist ferner zu erkennen, dass der letzte Ring 28 einen proximalen Spitzbogen 33 - und dadurch auch einen distalen Spitzbogen 34 - weniger aufweist als der vorletzte Ring 29 und als der Ring 23. Bei dieser Ausführungsform fehlt demnach im Vergleich zu Fig. 2 der distale Spitzbogen 39 (siehe Fig. 2).

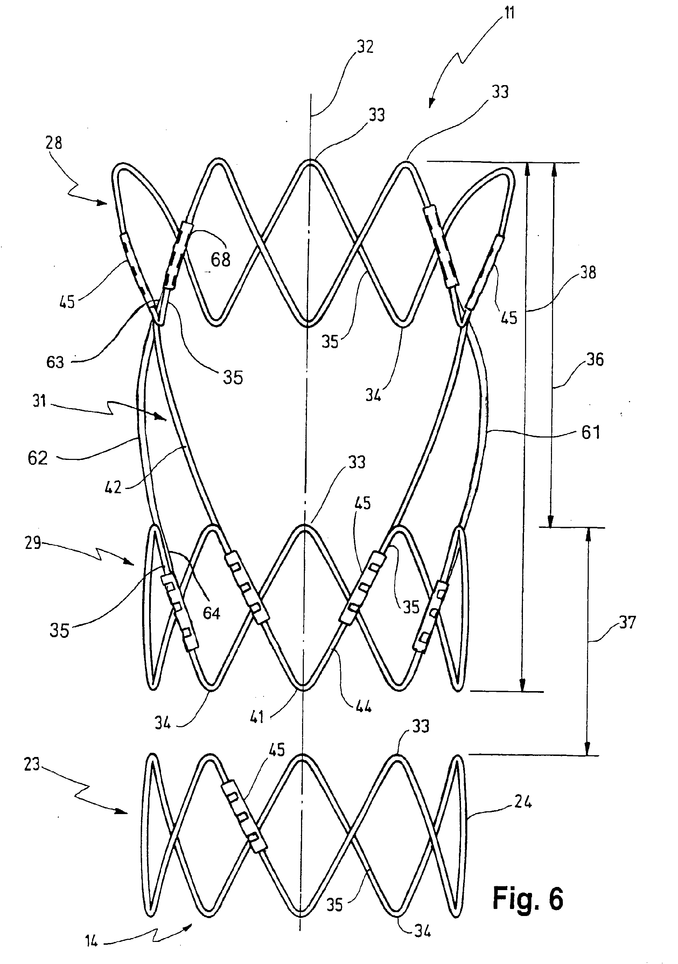

In Fig. 6 ist eine weitere Ausführungsform des erfindungsgemäßen Stents dargestellt, wobei diese Ausführungsform im Vergleich zur Ausführungsform aus Fig. 2 zusätzliche Verbindungsstützen 61 und 62 aufweist. Die Verbindungsstützen 61 und 62 sind mit ihrem proximalen Ende 63 jeweils mit einem Stützabschnitt 35 des letzten Ringes 28 und mit ihrem distalen Ende 64 jeweils mit einem Stützabschnitt 35 des vorletzten Ringes 29 in Anlage gebracht. In der Fig. 6 ist zu erkennen, dass dadurch die zusätzlichen Verbindungsstützen 61 und 62 eine Art Flankenverstärkung für den Stent darstellen.

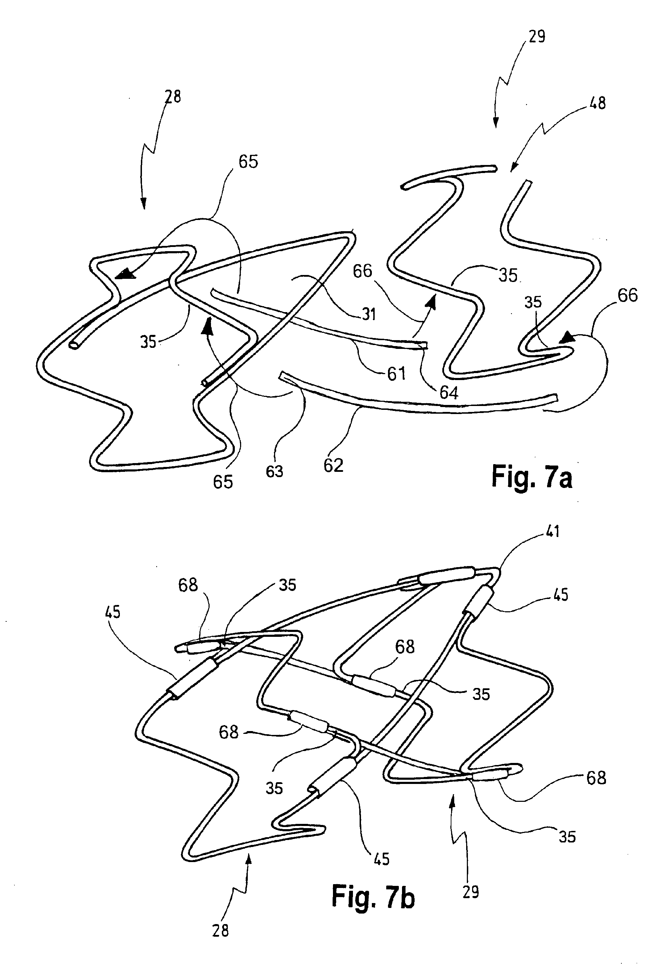

In Fig. 7a sind der letzte Ring 28 sowie der vorletzte Ring 29 unmittelbar nach der Fertigung, also noch vor der Verbindung miteinander, dargestellt. In Fig. 7a ist - wie in Fig. 3 - zu erkennen, dass der letzte Ring 28 und die Verbindungsstütze 31 einstückig miteinander ausgebildet sind. Die zusätzlichen Verbindungsstützen 61 und 62 werden mit ihrem proximalen Ende 63 mit einem Stützabschnitt 35 des letzten Ringes 28 in Anlage gebracht, was durch die Pfeile 65 angedeutet ist, sowie die distalen Enden 64 jeweils mit einem Stützabschnitt 35 des vorletzten Ringes 29, was durch die Pfeile 66 angedeutet ist.

In Fig. 7b ist zu erkennen, dass die beiden Ringe 28 und 29 zusammengeführt sind, wobei die Enden jeweils über Presshülsen 68 mit den entsprechenden Stützabschnitten 35 verbunden sind, so dass Verletzungen der Aorteninnenwand vermieden werden.

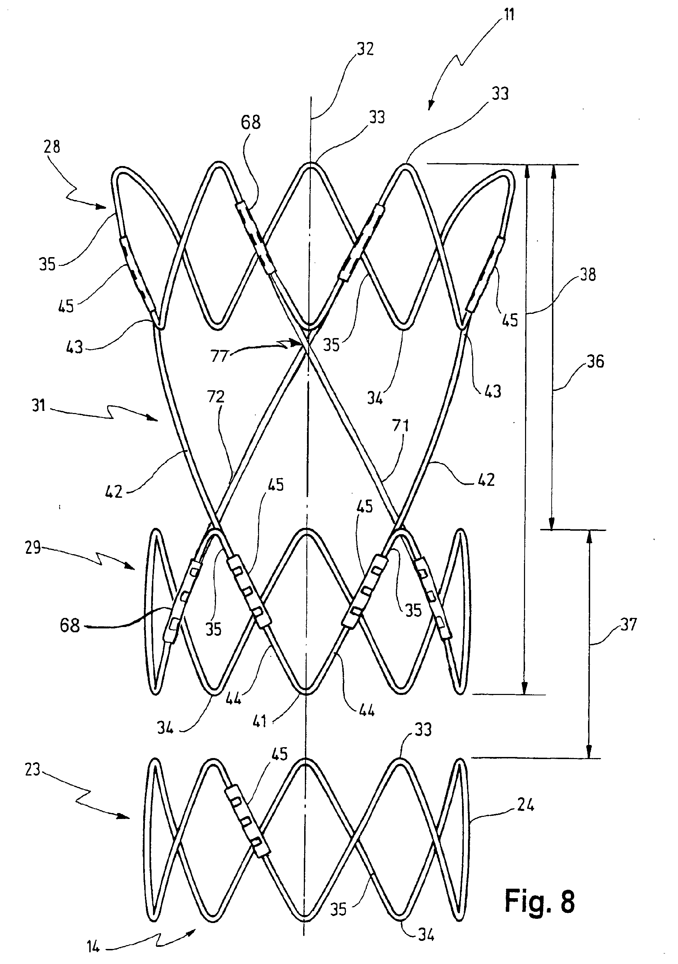

In Fig. 8 ist noch eine weitere Ausführungsform des erfindungsgemäßen Stents dargestellt, wobei hier wie in Fig. 6 ebenfalls zwei zusätzliche Verbindungsstützen 71 und 72 vorgesehen sind, die sich aber in einem Punkt 77 kreuzen.

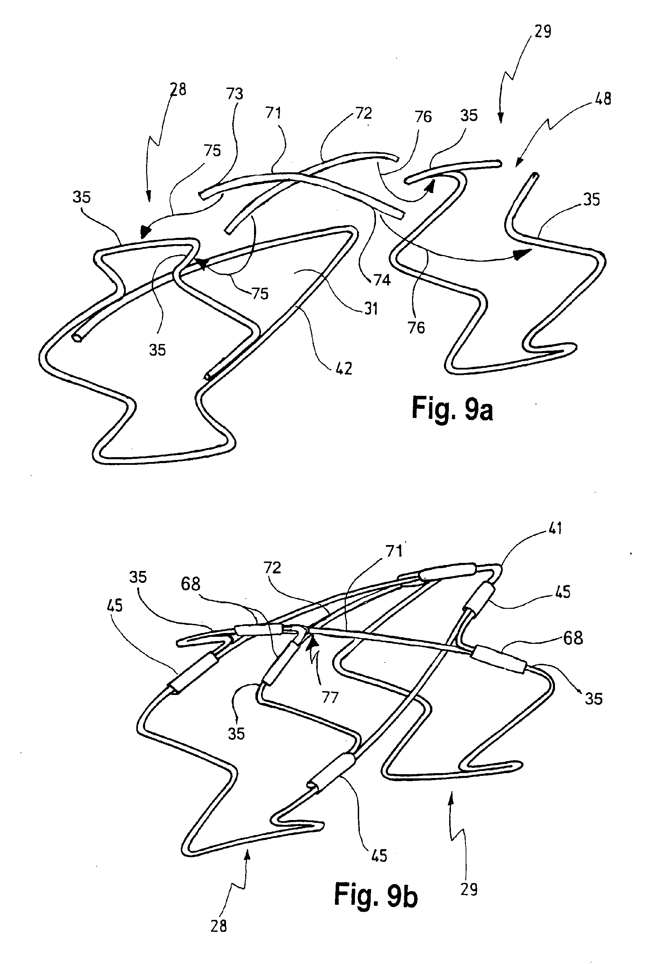

Ähnlich wie in Fig. 7a ist in Fig. 9a zu erkennen, dass die sich kreuzenden Verbindungsstützen 71 und 72 jeweils über ihr proximales Ende 73 mit einem Stützabschnitt 35 des letzten Ringes 28 in Anlage gebracht werden, was durch die Pfeile 75 angedeutet ist, und mit ihrem distalen Ende 74 mit einem Stützabschnitt 35 des vorletzten Ringes 29, was mit den Pfeilen 76 angedeutet ist.

In Fig. 9b ist gezeigt, zu erkennen, dass die beiden Ringe 28 und 29 zusammengeführt sind, wobei die Enden der Verbindungsstützen 71 und 72 durch Anbringen von Presshülsen 68 mit den jeweiligen Stützabschnitten 35 des letzten Ringes 28 bzw. des vorletzten Ringes 29 verbunden werden.

## Patentansprüche

1. Stent zur Implantation in ein Blutgefäß (15), insbesondere im Bereich des Aortenbogens (12), mit in seiner Längsrichtung (32) hintereinander angeordneten Ringen (23, 28, 29) aus mäanderförmig umlaufenden Stützen (24) und einem an den Ringen (23, 28, 29) befestigten und diese verbindenden Prothesenmaterial (25), das einen hohlzylindrischer Körper mit umfänglich im Wesentlichen geschlossenen Mantel (21) bildet,
**dadurch gekennzeichnet, dass** nur zwischen dem letzten (28) und dem vorletzten (29) Ring am proximalen Ende (14) des Stents (10) zumindest eine V-förmige Verbindungsstütze (31) mit zwei Schenkeln (42), die sich V-förmig zum distalen Ende (11) des Stents (10) erweitern, vorgesehen ist, wobei die V-förmige Verbindungsstütze (31) den letzten (28) und den vorletzten (29) Ring miteinander verbindet, und dass die Ringe im distalen Bereich lediglich über das Prothesenmaterial (25) miteinander verbunden sind.

2. Stent nach Anspruch 1, **dadurch gekennzeichnet, dass** zwischen dem letzten und dem vorletzten Ring (28, 29) im Bereich der Verbindungsstütze (31) ein im Wesentlichen von Prothesenmaterial (25) freier Mantelbereich (27) aufgespannt ist.

3. Stent nach Anspruch 1 oder 2, **dadurch gekennzeichnet, dass** der im Wesentlichen von Prothesenmaterial (25) freie Mantelbereich (27) sich zum proximalen Ende (11) des Stents (10) keilförmig aufweitet.

4. Stent nach einem der Ansprüche 1 bis 3, **dadurch gekennzeichnet, dass** die Stützen (24) einen Z-förmigen Verlauf mit abwechselnd zum proximalen (11) und zum distalen (14) Ende des Stents (10) weisenden Spitzbögen (33, 34) aufweisen, die durch schräg zur Längsrichtung (32) verlaufende Stützabschnitte (35) miteinander verbunden sind.

5. Stent nach Anspruch 4, **dadurch gekennzeichnet, dass** die proximalen Spitzbögen (33) des letzten Ringes (28) zu den proximalen Spitzbögen (33) des vorletzten Ringes (29) einen Abstand (36) aufweisen, der größer ist als der Abstand (37) zwischen den proximalen Spitzbögen (33) des vorletzten Ringes (29) und den proximalen Spitzbögen (33) des vorvorletzten Ringes (23).

6. Stent nach Anspruch 4 oder 5, **dadurch gekennzeichnet, dass** die proximalen Spitzbögen (33) des letzten Ringes (28) zu den distalen Spitzbögen (34) des vorletzten Ringes (29) eines Abstand (38) aufweisen, der größer ist als der Abstand (38) zwischen dem Abgang der A. subclavia sinister (19) und dem Abgang der A. carotis communis (18) vom Aortenbogen (12).

7. Stent nach einem der Ansprüche 4 bis 6, **dadurch gekennzeichnet, dass** ein zum distalen Ende (14) des Stents (10) weisender Spitzbogen (41) des vorletzten Ringes (29) mit einem Spitzbogen der Verbindungsstütze (31) in Anlage ist.

8. Stent nach Anspruch 7, **dadurch gekennzeichnet, dass** der zum distalen Ende (14) des Stents (10) weisende Spitzbogen (41) des vorletzten Ringes (29) zumindest teilweise durch den Spitzbogen der Verbindungsstütze (31) gebildet ist.

9. Stent nach einem der Ansprüche 4 bis 8, **dadurch gekennzeichnet, dass** die zwei Schenkel (42) der Verbindungsstütze (31) an ihrem proximalen Ende (43) jeweils mit einem Stützabschnitt (35) des letzten Ringes (28) in Anlage sind.

10. Stent nach einem der Ansprüche 4 bis 9, **dadurch gekennzeichnet, dass** die zwei Schenkel (42) der Verbindungsstütze (31) an ihrem distalen Ende (44) jeweils mit einem Stützabschnitt (35) des vorletzten Ringes (29) in Anlage sind.

11. Stent nach einem der Ansprüche 1 bis 10, **dadurch gekennzeichnet, dass** die Anzahl der Spitzbögen (33; 34) des letzten Ringes (28) geringer ist als die Anzahl der Spitzbögen (33, 34) des vorletzten Ringes (29)

12. Stent nach einem der Ansprüche 4 bis 10, **dadurch gekennzeichnet, dass** zumindest zwei weitere Verbindungsstützen (61, 62; 71, 72) vorgesehen sind, die mit ihrem proximalen Ende (63) jeweils mit einem Stützabschnitt (35) des letzten Ringes (28) und mit ihrem distalen Ende (64) jeweils mit einem Stützabschnitt (35) des vorletzten Ringes (29) in Anlage sind.

13. Stent nach Anspruch 12, **dadurch gekennzeichnet, dass** die Verbindungsstützen (71, 72) über ihr jeweils proximales Ende (73) und jeweils distales Ende (74) mit den Stützabschnitten (35) des letzten (28) und vorletzten (29) Ringes derart in Anlage sind, dass sich die Verbindungsstützen (71, 72) in einem Punkt (77) kreuzen.

14. Stent nach einem der Ansprüche 1 bis 13, **dadurch gekennzeichnet, dass** der letzte Ring (28) und die Verbindungsstütze (31) einstückig miteinander ausgebildet sind.

15. Stent nach einem der Ansprüche 4 bis 14, **dadurch gekennzeichnet, dass** der jeweilige Stützabschnitt (35) des letzten (28) oder vorletzten (29) Ringes und ein mit diesem in Anlage befindlicher Abschnitt der zumindest einen Verbindungsstütze (31; 61; 62; 71, 72) durch eine Presshülse (45; 68) miteinander verbunden sind.

16. Stent nach einem der Ansprüche 4 bis 15, **dadurch gekennzeichnet, dass** die zum proximalen Ende (11) des Stents (10) weisenden Spitzbögen (33) des letzten Ringes (28) gegenüber dessen zum distalen Ende (14) des Stents (10) weisenden Spitzbögen (34) nach außen gebogen sind.

17. Stent nach einem der Ansprüche 1 bis 16, **dadurch gekennzeichnet, dass** die Stützen (24) und die zumindest eine Verbindungsstütze (31; 61, 62; 71; 72) aus einem drahtförmigen, elastischen Material bestehen.

18. Stent nach einem der Ansprüche 1 bis 17, **dadurch gekennzeichnet, dass** distal zu einem distalen Spitzbogen (41) der Verbindungsstütze (31) an dem Mantel (26) ein distaler Marker (46) angeordnet ist.

19. Stent nach einem der Ansprüche 4 bis 18, **dadurch gekennzeichnet, dass** distal zu einem Stützabschnitt (35) des letzten Ringes (28) an dem Mantel (26) ein proximaler Marker (47) angeordnet ist.

20. Stent nach Anspruch 18 oder 19, **dadurch gekennzeichnet, dass** der Marker (46, 47) ein Röntgenmarker ist.

21. Stent nach einem der Ansprüche 1 bis 20, **dadurch gekennzeichnet, dass** das Prothesenmaterial (25) aus textilem Material oder aus Folie besteht.

22. Stent nach einem der Ansprüche 1 bis 21, **dadurch gekennzeichnet, dass** das Prothesenmaterial (25) an den Stützen (14) und der zumindest einen Verbindungsstütze (31; 61, 62; 71, 72) durch Nähen, Kleben oder Einschmelzen befestigt ist.

## Claims

1. A stent for implantation in a blood vessel (15), especially in the region of the aortic arch (12), with rings (23, 28, 29) which are disposed successively in its longitudinal direction (32) and which are made up of meandering circumferential supports (24), and with a prosthesis material (25) which is fixed to the rings (23, 28, 29) and which connects them, said prosthesis material (25) forming a hollow cylindrical body with a jacket (21) which is substantially closed on the circumference thereof,
**characterized in that** only between the last ring (28) and the penultimate ring (29) at the proximal end (14) of the stent (10) at least one V-shaped connecting support (31) comprising two legs (42) is provided, which two legs (42) widen in a V-shape to the distal end (11) of the stent (10), wherein the V-shaped connecting support (31) connects the last ring (28) and the penultimate ring (29) at the proximal end (14) of the stent to one another, and **in that** in the distal area the rings are connected to one another by the prosthesis material (25) solely.

2. The stent as claimed in claim 1, **characterized in that** a jacket area (27) substantially free of prosthesis material (25) is braced between the last ring (28) and the penultimate ring (29) in the area of the connecting support (31).

3. The stent as claimed in claim 1 or 2, **characterized in that** the jacket area (27) substantially free of prosthesis material (25) widens in a wedge shape toward the proximal end (11) of the stent (10).

4. The stent as claimed in one of claims 1 to 3, **characterized in that** the supports (24) have a Z-shaped profile with pointed arches (33, 34) pointing alternately toward the proximal end (11) and distal end (14) of the stent (10), which pointed arches (33, 34) are connected to one another by support portions (35) extending obliquely with respect to the longitudinal direction (32).

5. The stent as claimed in claim 4, **characterized in that** the proximal pointed arches (33) of the last ring (28) are spaced apart from the proximal pointed arches (33) of the penultimate ring (29) by a distance (36) which is greater than the distance (37) between the proximal pointed arches (33) of the penultimate ring (29) and the proximal pointed arches (33) of the third last ring (23).

6. The stent as claimed in claim 4 or 5, **characterized in that** the proximal pointed arches (33) of the last ring (28) are spaced apart from the distal pointed arches (34) of the penultimate ring (29) by a distance (38) which is greater than the distance (38) between the origin of the left subclavian artery (19) and the origin of the common carotid artery (18) from the aortic arch (12).

7. The stent as claimed in one of claims 4 to 6, **characterized in that** a pointed arch (41) of the penultimate ring (29) pointing toward the distal end (14) of the stent (10) is in contact with a pointed arch of the connecting support (31).

8. The stent as claimed in claim 7, **characterized in that** the pointed arch (41) of the penultimate ring (29) pointing toward the distal end (14) of the stent (10) is formed at least partially by the pointed arch (1) of the connecting support (31).

9. The stent as claimed in one of claims 4 to 9, **characterized in that** the two legs (42) of the connecting support (31) at their proximal end (43) are each in contact with a support portion (35) of the last ring (28).

10. The stent as claimed in one of claims 4 to 9, **characterized in that** the two legs (42) of the connecting support (31) at their distal end (44) are each in contact with a support portion (35) of the penultimate ring (29).

11. The stent as claimed in one of claims 1 to 10, **characterized in that** the number of pointed arches (33; 34) of the last ring (28) is smaller than the number of pointed arches (33, 34) of the penultimate ring (29).

12. The stent as claimed in one of claims 4 to 10, **characterized in that** at least two further connecting supports (61, 62; 71, 72) are provided which, with their proximal end (63), are each in contact with a support portion (35) of the last ring (28) and which, with their distal end (64), are each in contact with a support portion (35) of the penultimate ring (29).

13. The stent as claimed in claim 12, **characterized in that** the connecting supports (71, 72) are in contact via their respective proximal end (73) and respective distal end (74) with the support portions (35) of the last ring (28) and penultimate ring (29) in such a way that the connecting supports (71, 72) intersect at a point (77).

14. The stent as claimed in one of claims 1 to 13, **characterized in that** the last ring (28) and the connecting support (31) are formed integrally with one another.

15. The stent as claimed in one of claims 4 to 14, **characterized in that** the respective support portion (35) of the last ring (28) or penultimate ring (29) and a portion of the at least one connecting support (31; 61, 62; 71, 72) situated in contact with said supporting portion (35) are connected to one another by a crimp sleeve (45; 68).

16. The stent as claimed in one of claims 4 to 15, **characterized in that** the pointed arches (33) of the last ring (28) which point toward the proximal end (11) of the stent (10) are bent outward in relation to its pointed arches (34) pointing toward the distal end (14) of the stent (10).

17. The stent as claimed in one of claims 1 to 16, **characterized in that** the supports (24) and the at least one connecting support (31; 61, 62; 71, 72) are made of a wire-like, elastic material.

18. The stent as claimed in one of claims 1 to 17, **characterized in that** a distal marker (46) is disposed on the jacket (26) at a position in the distal direction from a distal pointed arch (41) of the connecting support (31).

19. The stent as claimed in one of claims 4 to 18, **characterized in that** a proximal marker (47) is disposed on the jacket (26) at a position in the distal direction from a support portion (35) of the last ring (28).

20. The stent as claimed in claim 18 or 19, **characterized in that** the marker (46, 47) is an X-ray marker.

21. The stent as claimed in one of claims 1 to 20, **characterized in that** the prosthesis material (25) is composed of a textile material or of film.

22. The stent as claimed in one of claims 1 to 22, **characterized in that** the prosthesis material (25) is fixed on the supports (14) and on the at least one connecting support (31; 61, 62; 71, 72) by sewing, gluing or melting in.

## Revendications

1. Stent destiné à l'implantation dans un vaisseau sanguin (15), en particulier dans la région de la crosse de l'aorte (12), avec des anneaux (23, 28, 29) en soutiens périphériques sinueux (24) disposés l'un derrière l'autre dans sa direction longitudinale (32) et avec un matériau de prothèse (25) fixé aux anneaux (23, 28, 29) et reliant ceux-ci, qui forme un corps cylindrique creux avec une enveloppe (21) essentiellement fermée en périphérie, **caractérisé en ce qu'**il est prévu uniquement entre le dernier anneau (28) et l'avant-dernier anneau (29), à l'extrémité proximale (14) du stent (10), au moins un soutien de liaison en forme de V (31) avec deux branches (42), qui s'élargissent en forme de V vers l'extrémité distale (11) du stent (10), dans lequel le soutien de liaison en forme de V (31) relie l'un à l'autre le dernier anneau (28) et l'avant-dernier anneau (29), et **en ce que** les anneaux sont reliés l'un à l'autre dans la région distale uniquement par le matériau de prothèse (25).

2. Stent selon la revendication 1, **caractérisé en ce qu'**une région d'enveloppe (27) essentiellement exempte de matériau de prothèse (25) est tendue entre le dernier anneau (28) et l'avant-dernier anneau (29) dans la région du soutien de liaison (31).

3. Stent selon la revendication 1 ou 2, **caractérisé en ce que** la région d'enveloppe (27) essentiellement exempte de matériau de prothèse (25) s'élargit en forme de coin vers l'extrémité proximale (11) du stent (10).

4. Stent selon l'une quelconque des revendications 1 à 3, **caractérisé en ce que** les soutiens (24) présentent un tracé en forme de Z avec des courbes de pointe (33, 34) orientées alternativement vers l'extrémité proximale (11) et vers l'extrémité distale (14) du stent (10), qui sont reliées l'une à l'autre par des sections de soutien (35) orientées en oblique par rapport à la direction longitudinale (32).

5. Stent selon la revendication 4, **caractérisé en ce que** les courbes de pointe proximales (33) du dernier anneau (28) présentent par rapport aux courbes de pointe proximales (33) de l'avant-dernier anneau (29) une distance (36) qui est plus grande que la distance (37) entre les courbes de pointe proximales (33) de l'avant-dernier anneau (29) et les courbes de pointe proximales (33) de l'antépénultième anneau (23).

6. Stent selon la revendication 4 ou 5, **caractérisé en ce que** les courbes de pointe proximales (33) du dernier anneau (28) présentent par rapport aux courbes de pointe distales (34) de l'avant-dernier anneau (29) une distance (38) qui est plus grande que la distance (38) entre le départ de A. subclavia sinistra (19) et le départ de A. carotis communis (18) à partir de la crosse de l'aorte (12).

7. Stent selon l'une quelconque des revendications 4 à 6, **caractérisé en ce qu'**une courbe de pointe (41) de l'avant-dernier anneau (29) orientée vers l'extrémité distale (14) du stent (10) est appuyée sur une courbe de pointe du soutien de liaison (31).

8. Stent selon la revendication 7, **caractérisé en ce que** la courbe de pointe (41) de l'avant-dernier anneau (29) orientée vers l'extrémité distale (14) du stent (10) est formée au moins en partie par la courbe d'extrémité du soutien de liaison (31).

9. Stent selon l'une quelconque des revendications 4 à 8, **caractérisé en ce que** les deux branches (42) du soutien de liaison (31) sont appuyées à leur extrémité proximale (43) respectivement sur une section de soutien (35) du dernier anneau (28).

10. Stent selon l'une quelconque des revendications 4 à 9, **caractérisé en ce que** les deux branches (42) du soutien de liaison (31) sont appuyées à leur extrémité distale (44) respectivement sur une section de soutien (35) de l'avant-dernier anneau (29).

11. Stent selon l'une quelconque des revendications 1 à 10, **caractérisé en ce que** le nombre des courbes de pointe (33; 34) du dernier anneau (28) est inférieur au nombre des courbes de pointe (33, 34) de l'avant-dernier anneau (29).

12. Stent selon l'une quelconque des revendications 4 à 10, **caractérisé en ce qu'**il est prévu au moins deux autres soutiens de liaison (61, 62; 71, 72), qui sont appuyés par leur extrémité proximale (63) respectivement sur une section de soutien (35) du dernier anneau (28) et par leur extrémité distale (64) respectivement sur une section de soutien (35) de l'avant-dernier anneau (29).

13. Stent selon la revendication 12, **caractérisé en ce que** les soutiens de liaison (71, 72) sont appuyés par leur extrémité respectivement proximale (73) et leur extrémité respectivement distale (74) sur les sections de soutien (35) du dernier anneau (28) et de l'avant-dernier anneau (29), de telle manière que les soutiens de liaison (71, 72) se croisent en un point (77).

14. Stent selon l'une quelconque des revendications 1 à 13, **caractérisé en ce que** le dernier anneau (28) et le soutien de liaison (31) sont réalisés d'une seule pièce l'un avec l'autre.

15. Stent selon l'une quelconque des revendications 4 à 14, **caractérisé en ce que** la section de soutien respective (35) du dernier anneau (28) ou de l'avant-dernier anneau (29) et une section se trouvant en appui sur celui-ci dudit au moins un soutien de liaison (31; 61; 62; 71, 72) sont assemblées l'une à l'autre par un manchon comprimé (45; 68).

16. Stent selon l'une quelconque des revendications 4 à 15, **caractérisé en ce que** les courbes de pointe (33) du dernier anneau (28) orientées vers l'extrémité proximale (11) du stent (10) sont courbées vers l'extérieur par rapport à ses courbes de pointe (34) orientées vers l'extrémité distale (14) du stent (10).

17. Stent selon l'une quelconque des revendications 1 à 16, **caractérisé en ce que** les soutiens (24) et ledit au moins un soutien de liaison (31; 61, 62; 71; 72) sont constitués d'un matériau élastique en forme de fil.

18. Stent selon l'une quelconque des revendications 1 à 17, **caractérisé en ce qu'**un marqueur distal (46) est disposé sur l'enveloppe (26) en position distale par rapport à une courbe de pointe distale (41) du soutien de liaison (31).

19. Stent selon l'une quelconque des revendications 4 à 18, **caractérisé en ce qu'**un marqueur proximal (47) est disposé sur l'enveloppe (26) en position distale par rapport à une section de soutien (35) du dernier anneau (28).

20. Stent selon la revendication 18 ou 19, **caractérisé en ce que** le marqueur (46, 47) est un marqueur à rayons X.

21. Stent selon l'une quelconque des revendications 1 à 20, **caractérisé en ce que** le matériau de prothèse (25) se compose de matériau textile ou de film.

22. Stent selon l'une quelconque des revendications 1 à 21, **caractérisé en ce que** le matériau de prothèse (25) est fixé aux soutiens (14) et audit au moins un soutien de liaison (31; 61, 62; 71, 72) par couture, collage ou fusion.
